# EUROPEAN PATENT APPLICATION

(11) **EP 2 319 457 A1**
(43) Date of publication of application: **11.05.2011**
(21) Application number: 09425453.9
(22) Date of filing: 10.11.2009
(51) Int. Cl.: A61F 2/16

(54) **Vision aiding system comprising at least one intraocular dioptric lens**

(71) Applicant: Lenspecial S.r.L., 20122 Milano (IT)
(72) Inventor: Podini, Remo, 20146 Milano (IT); Savaresi, Gaetano, 20093 Cologno Monzese (MI) (IT)
(74) Representative: Ripamonti, Enrico

(57) **Abstract**

The vision aid system according to the invention comprises at least one intraocular dioptric body (17) designed to be implanted in the eye of a user. The dioptric body comprises an optically active portion designed to project images onto the retina, if necessary interacting with the other parts of the vision aid system. The optically active portion comprises a central optical portion (17A) and a peripheral optical portion (17B) which extends around the central optical portion (17A). The central optical portion (17A) can form a negative lens and the peripheral optical portion (17B) can form a positive lens. When the user requires greater visual sharpness, the central optical portion (17A) interacts with the external lenses (19) such as spectacle lenses, for example. The external lenses (19) compensate in a non-invasive manner for visual dysfunctions occurring after implantation of a first intraocular lens, with which the intraocular dioptric body (17) can be combined if necessary.

## Description

### Field of the invention

The present invention concerns a vision aid system comprising at least one intraocular dioptric body, and a method for improving the visual function of a patient via the implant of at least one intraocular dioptric body.

### State of the art

For the treatment of some eye pathologies, the so-called IOL, IntraOcular Lenses, i.e. lenses designed to be implanted in the eye, are currently known. For example, for the treatment of cataract the natural crystalline lens is replaced with an artificial intraocular lens inserted in front of the iris - i.e. in the anterior chamber of the eye - or at the back of the iris, i.e. in the posterior chamber of the eye.

After the insertion of a first intraocular lens to treat a certain type of pathology, new problems sometimes arise which make said first lens inadequate and require the modification, replacement or integration thereof with other lenses.

IOL insertion techniques have developed over time and have become gradually less traumatic and invasive, for example due to the invention of IOLs which are inserted rolled up and then unroll inside the bulb, thus involving increasingly smaller incisions in the bulb. On the other hand, the removal of an intraocular lens from the bulb is a very problematic operation, which is avoided as far as possible.

A first object of the present invention is therefore to provide a vision aid system and a method for improving the visual capabilities of a patient by means of one or more intraocular dioptric bodies, which compensate more easily and in a less invasive manner for any visual dysfunctions not diagnosed or encountered when a first intraocular lens is implanted, and which occur or become manifest in the patent after said first implant, sometimes after several months or years.
A second object is to provide an alternative to the vision aid systems of known type, to remedy or at least partially compensate for poor lateral vision, such as tunnel vision, for example.

### Summary of the invention

Said objects are achieved, according to a first aspect of the present invention, with a vision aid system having the characteristics according to claim 1. In a second aspect of the present invention, said objects are achieved with a method for improving the visual function having the characteristics according to claim 13.

The advantages that can be obtained with the present invention will become more evident, to a person skilled in the art, from the following detailed description of some particular examples of non-limiting embodiment, illustrated with reference to the following schematic figures.

### List of the Figures

Figure 1 shows, in section according to an anterior-posterior plane, the eye of a patient to which a first embodiment of a vision aid system according to the present invention is applied;
Figure 2 shows a lateral section view of an intraocular lens of the system of Figure 1;
Figure 3 shows a frontal view of the intraocular lens of Figure 2;
Figure 4 shows, in section according to an anterior-posterior plane, the eye of a patient to which a second embodiment of a vision aid system according to the present invention is applied;
Figure 5 shows a lateral view in section of an intraocular lens according to a third embodiment of the vision aid system according to the present invention;
Figure 6 shows a lateral view in section of an intraocular prism according to a fourth embodiment of the vision aid system according to the present invention.

### Detailed description

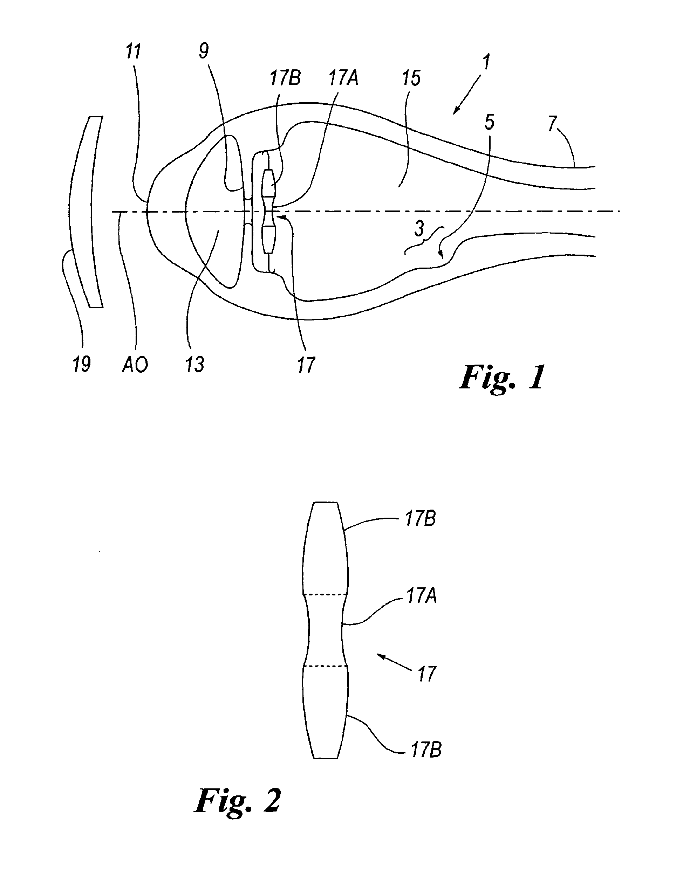

Figures 1-3 relate to a first embodiment of the vision aid system according to the invention.
Reference number 1 indicates an overview of a human eye. The eye 1 comprises the retina 3 and the macula 5, formed of a hollow portion of the retina richer in photoreceptors.
Reference number 6 indicates the macular edge, which delimits the macula. The optic nerve 7 starts from the posterior part of the eye 1, while the iris 9 and the cornea 11 are in the anterior part.

Between the iris and the cornea there is a first space -called anterior chamber 13- full of aqueous humour.
Behind the iris is a second space full of aqueous humour called posterior chamber 15.
The eye 1 shown belongs to an aphakic person, i.e. a person whose crystalline lens has been removed, therefore the sac which contained the crystalline is absent.

The vision aid system according to the embodiment of Figures 1-3 comprises an intraocular dioptric body 17 and an external lens 19; more in particular the dioptric body 17 is an intraocular lens.

The intraocular lens 17 replaces the natural crystalline lens of the patient and is inserted in the posterior chamber 15, for example fixed to the capsular sac or in the sulcus of an aphakic patient by means of the fixing peduncles 170, or other fixing systems known per se and not shown.

In this embodiment of the invention, the intraocular dioptric body 17 is provided with a negative central portion 17A which forms a negative or diverging lens, and peripheral portion 17B which surrounds the central portion 17A and forms a positive or converging lens.
Preferably the central portion 17A has a diameter DC of between 0.5 and 3 mm. Preferably the peripheral portion 17B has an external diameter DP between 5 and 7 mm, more preferably between 5 and 6 mm.
Preferably one or both the faces of the central portion 17A and the peripheral portion 17B comprise a portion of spherical surface having radius of curvature equal to or below 30 mm, according to the dioptres required of the portions 17A and 17B, independently of each other.
Preferably the central portion 17A comprises a biconcave lens, preferably spherical. Preferably the peripheral portion 17B comprises a biconvex lens, preferably spherical; said form in fact reduces insertion errors and is easier to produce.
Preferably but not necessarily the two portions 17A and 17B form lenses which are substantially axialsymmetric with respect to their common optical axis AO.

The external lens 19 can be for example a normal positive lens for spectacles, obviously having sufficient dioptres. In the embodiment of Figure 1 said lenses 19 are mounted on ordinary spectacle frames.
The intraocular lens 17 is designed to be used without or with the aid of the external lens 19.

Operation of the above-mentioned vision aid system is as follows.
When the user is not wearing glasses, the peripheral portion 17B of each intraocular lens 17 focuses the non-enlarged images IM on the retina RT: in this way a user who, for example, due to a macular dysfunction, has defective vision in the centre of the visual field is nevertheless able to perceive the images via his lateral vision; without perfectly distinguishing the details or having particularly sharp sight, the patient is able to perceive the objects in front of him and walk independently.
When the user needs to distinguish the details more clearly, he will wear glasses or other external lenses 19: the unit formed by each external lens 19 and the negative central portion 17A of the corresponding intraocular lens 17 constitutes a converging optical system which focuses the enlarged images IM' on the retina RT; in this way the user is able to capture, again via lateral vision, the enlarged details of the images, improving in percentage terms his visual sharpness.

One advantage of the system according to the invention is that the external lenses 19 can be changed and adapted very easily and without surgery to changes in patient needs, for example to correct vision defects absent at the time of implantation of the intraocular lens 17 and occurring subsequently, whereas the replacement of an intraocular lens with another one would be a much more complex, invasive and risky operation for the patient, and may even be impossible. The external lenses 19 can if necessary incorporate any refractive residue or be made for different vision distances.

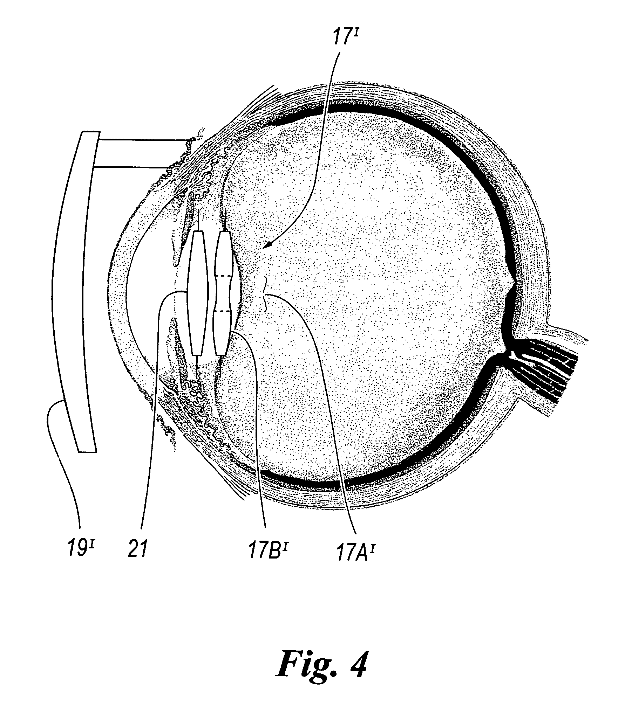

Figure 4 shows a second embodiment of a vision aid system according to the invention. Said system also comprises an additional intraocular lens 17' and an external lens 19'.
The additional intraocular lens 17' is designed to be implanted in the patient's eye, preferably in the posterior chamber 15, in addition to another intraocular lens 21 implanted previously, in front of or behind the existing lens 21.
The unit comprising the two - or if necessary several - intraocular lenses 17' and 21 replaces the patient's natural crystalline lens.

Also the additional intraocular lens 17' comprises a negative central portion 17A' which forms a negative or diverging lens, and a peripheral portion 17B' which surrounds the central portion 17A' and, as required, can form a positive or converging lens or be simply an optically neutral portion, for example a refractive wall of substantially constant thickness. Generally, all other conditions being equal, the portions 17A' and 17B' will have different powers and/or dioptres with respect to the embodiment of Figures 1-3.
Preferably but not necessarily the two portions 17A' and 17B' form lenses which are substantially axialsymmetric with respect to their common optical axis AO.

Operation of the vision aid system of Figure 4 is analogous to that of the system of Figure 1, with the difference that in the system of Figure 4 the unit formed by the two intraocular lenses 17' and 21 replaces the single intraocular lens 17 of Figure 1.

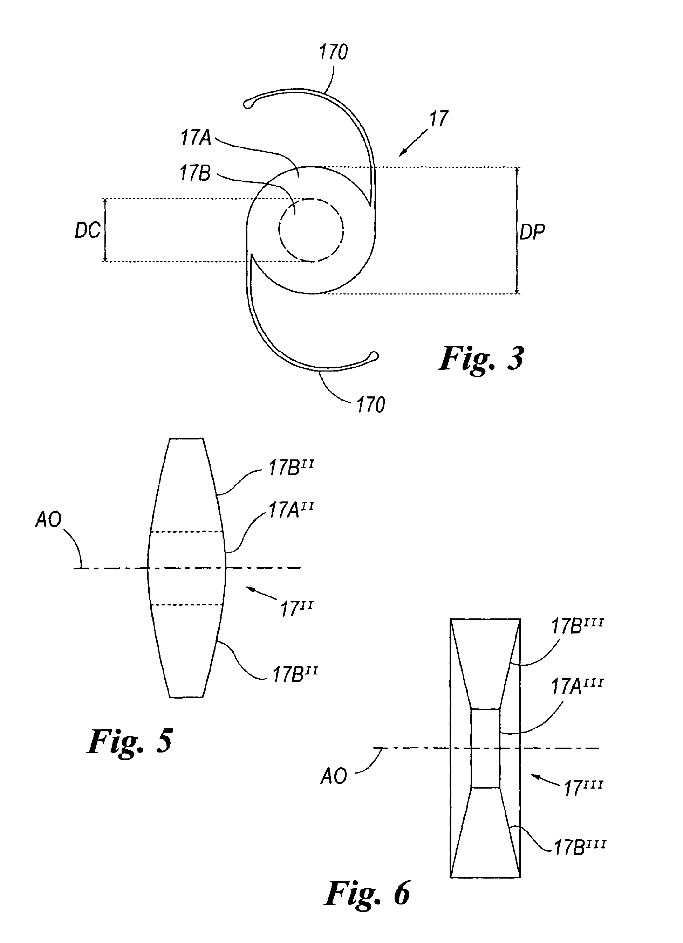

Figure 5 shows a third embodiment of a vision aid system according to the invention, particularly suitable for persons who do not have problems seeing the details in the centre of their field of vision but have peripheral vision problems, although without having a much more restricted field of vision than a normal person. Said system comprises an intraocular lens 17" and an external lens 19'. The intraocular lens 17" comprises a neutral central portion 17A" and a peripheral portion 17B" which surrounds the central portion 17A and substantially forms a positive or converging lens. The neutral central portion 17A" does not constitute a lens and does not vary the convergence or divergence of the rays of light that cross it, and can be formed for example of a wall of substantially transparent material and substantially constant thickness, for example flat, or of a through hole.
The diameters of the central portion 17A" and peripheral portion 17B" can be chosen with the same criteria as the embodiments of Figures 1-3; preferably the central portion 17A" has a diameter of between 0.3 mm and 1.5 mm.

When the patient is not wearing glasses or other external lenses 19, 19', he simply sees through the central portion 17A" as through a hole.
The external lenses 19, 19' are preferably negative and, when the patient wears them, the combination of the peripheral portion 17B" and the negative external lenses 19, 19' produces a sort of wide-angle optical system which, like the peephole of a door, reduces, inside the central part of the field of vision, an angularly larger portion of space, allowing him to see with the vision defined the periphery of the scene before him, which a normal person would see with his lateral vision.
With this embodiment it is possible to enlarge the patient's field of vision up to approximately 30% symmetrically around the optical axis AO.

Figure 6 shows a fourth embodiment of a vision aid system according to the invention, particularly suitable for persons who do not have problems seeing the details in the centre of their field of vision but have serious problems of peripheral vision, such as tunnel vision, for example. Said system can comprise even only one intraocular dioptric body 17"' for use without glasses or other external lenses. The intraocular dioptric body 17"' comprises an optically neutral central portion 17A"' and a peripheral portion 17B"' which surrounds the central portion 17A"' and substantially forms a so-called diverging annular prism, i.e. a toroidal-shaped refractive solid, the generatrix surface of which is a trapezium, and the thickness of which diminishes the nearer it gets to the optical axis AO.
The optically neutral central portion 17A" can be analogous to that of the intraocular lens 17" of Figure 5.
The diameters of the central portion 17A"' and peripheral portion 17B"' can be chosen with the same criteria as the embodiments of Figures 1-3.
The patient is able to see the details of the images, perceiving them with the central area of his field of vision; said portion of the field of vision reaches the retina by simply crossing the neutral central portion 17A"'. The peripheral portion 17B"', on the other hand, functions substantially as a wide-angle lens or the peephole of a door, introducing into the field of vision an angularly larger portion of space allowing the patient to see, with the vision defined, the periphery of the scene before him, which a normal person would see via his lateral vision. In other words the intraocular dioptric body 17"' increases the angular aperture of the person's field of vision. Currently no cures or prostheses are known to remedy or alleviate the drawbacks of tunnel vision.

The embodiment examples previously described are subject to various modifications and variations without departing from the protective scope of the present invention. For example, in an embodiment not shown, each external lens 19 can be produced as a contact lens instead of a spectacle lens.

The examples and lists of possible variations of the present application are not exhaustive.

## Claims

1. Vision aid system comprising at least one intraocular dioptric body (17, 17', 17", 17"') designed for implantation in the eye of a user and comprising an optically active portion designed to project images onto the retina, if necessary interacting with the other parts of the vision aid system, wherein:
- the optically active portion comprises a central optical portion (17A, 17A', 17A", 17A"') and a peripheral optical portion (17B, 17B', 17B", 17B"') which extends around the central optical portion (17A, 17A', 17A", 17A"');
- the central optical portion (17A, 17A', 17A", 17A"') can substantially form a negative lens or an optically neutral portion, i.e. which is different from a lens and does not vary the convergence or the divergence of the light rays that cross it;
- if the central optical portion (17A, 17A', 17A", 17A"') substantially forms a negative lens, the peripheral optical portion (17B, 17B', 17B", 17B"') substantially forms a positive lens or an optically neutral portion;
- if the central optical portion (17A, 17A', 17A", 17A"') substantially forms an optically neutral portion, the peripheral optical portion (17B, 17B', 17B", 17B"') substantially forms a positive lens or a substantially annular prism with increasing thickness as it moves radially away from the centre towards the periphery of the ring.

2. Vision aid system as claimed in claim 1, comprising an external lens (19) designed to be worn by the user on the outside of the eye in which the intraocular dioptric body is implanted (17, 17', 17", 17"') and to cooperate with the intraocular dioptric body (17, 17', 17", 17"') in order to focus the images on the retina.

3. Vision aid system as claimed in claim 1, wherein the optically neutral portion (17A", 17A"') comprises a through hole or a substantially transparent wall of substantially constant thickness.

4. Vision aid system as claimed in claim 2, wherein the central optical portion (17A) forms a negative lens designed to cooperate with the external lens (19) in order to project enlarged images onto the retina of the eye.

5. Vision aid system as claimed in claim 2, wherein the peripheral optical portion (17B) is designed to focus on the retina the non-enlarged images at least when the user is not wearing the external lens (19).

6. Vision aid system as claimed in claim 1, wherein the peripheral optical portion (17B) has an external diameter (DP) of between 5 and 7 mm, and if necessary between 5 and 6 mm.

7. Vision aid system as claimed in claim 1, wherein the central optical portion (17A) has a diameter (DC) of between 0.5 and 3 mm.

8. Vision aid system as claimed in claim 2, wherein the external lens (19) is substantially a positive lens.

9. Vision aid system as claimed in claim 2, wherein:
- the external lens (19) is substantially a negative lens;
- the central optical portion (17A) forms an optically neutral portion such as a wall of substantially constant thickness or a through hole, for example;
- the peripheral optical portion (17B) forms a positive lens designed to cooperate with the external lens (19) in order to focus on the retina substantially reduced images captured with a visual angle greater than that of the intraocular dioptric body alone (17, 17').

10. Vision aid system as claimed in claim 1, wherein the central optical portion (17A) substantially forms an optically neutral portion and the peripheral optical portion (17B) substantially forms an annular prism increasing in thickness as it moves radially farther away from the centre towards the periphery of the ring, the prism being designed to project onto the retina images captured with a visual angle greater than that of a normal healthy eye without artificial intraocular dioptric bodies (17, 17').

11. Vision aid system as claimed in claim 1, comprising a pair of glasses on which at least one external lens (19) is mounted or a contact lens as external lens (19).

12. System as claimed in claim 1, wherein the at least one intraocular dioptric body (17) is designed to be implanted in the posterior chamber of the patient's eye, and/or behind the iris.

13. Method for improving the visual function of a patient, comprising the operation of implanting at least one intraocular dioptric body (17, 17') in the eye of a patient, wherein the intraocular dioptric body (17, 17') has the characteristics as claimed in claim 1.

14. Method as claimed in claim 13, comprising the operation of implanting the at least one intraocular dioptric body (17, 17') in the posterior chamber of the patient's eye and/or behind the iris of the patient's eye.

15. Method as claimed in claim 13, comprising the operation of surgically implanting the at least one first intraocular dioptric body (17, 17') in the patient's eye in addition to at least one second intraocular dioptric body (21), wherein:
- the at least one second intraocular dioptric body (21) has already been implanted in the patient's eye prior to surgery;
- the at least one first (17, 17') and at least one second intraocular dioptric body (21) optically cooperate in order to focus images on the retina, improving the visual capabilities of the patient.
